# FASCICULE DE BREVET EUROPEEN

(11) **EP 2 422 405 B1**
(45) Date de publication et mention de la délivrance du brevet: **27.01.2016**
(21) Numéro de dépôt: 10717602.6
(22) Date de dépôt: 23.04.2010
(51) Int. Cl.: H01R 4/50, H01R 13/52, H01R 13/58, A61N 1/375

(54) **DISPOSITIF DE CONNEXION ÉLECTRIQUE IMPLANTABLE DANS LE CORPS HUMAIN**
IN DEN MENSCHLICHEN KÖRPER IMPLANTIERBARE ELEKTRISCHE VERBINDUNGSEINRICHTUNG
ELECTRICAL CONNECTION DEVICE IMPLANTABLE IN THE HUMAN BODY

(30) Priorité: 23.04.2009 FR 0952659
(43) Date de publication de la demande: 29.02.2012
(73) Titulaire: Centre Hospitalier Universitaire de Rouen, 76000 Rouen (FR)
(72) Inventeur: SABIN, Pierre, F-75008 Paris (FR)
(74) Mandataire: Regimbeau
(86) Numéro de dépôt international: PCT/EP2010/055426
(87) Numéro de publication internationale: WO 2010/122140

(56) Documents cités:
- EP-A- 1 504 789
- WO-A-98/57702
- WO-A-03/092794
- US-A- 4 411 276
- US-A- 4 934 366
- US-A- 6 043 273

## Description

### DOMAINE DE L'INVENTION

La présente invention concerne les connexions électriques dans le domaine médical, et plus particulièrement un dispositif de connexion électrique implantable dans le corps d'un animal, en particulier dans le corps humain.

### ETAT DE LA TECHNIQUE

Il y a eu ces dernières années un développement important d'équipements électriques conçus pour être installés à l'intérieur du corps d'un patient. On peut citer de manière non limitative des dispositifs médicaux implantés pour pallier à une défaillance d'un organe naturel, tel que le coeur par exemple, des dispositifs ayant une fonction de contrôle de certains paramètres physiologiques, ou encore des dispositifs adaptés pour délivrer une dose particulière d'une substance thérapeutique particulière à un instant donné et dans une zone du corps spécifique.

Ce développement a été tel qu'il n'est pas rare que plusieurs dispositifs électriques médicaux soient implantés dans le corps d'un seul et même patient. Cela est par exemple le cas pour certains traitements qui doivent intervenir dans plusieurs zones distinctes, dans le cas où il est nécessaire d'avoir un dispositif électrique implanté pour superviser le fonctionnement d'autres dispositifs implantés, dans le cas où un dispositif d'alimentation électrique implanté alimente d'autres dispositifs implantés, etc.

Bien souvent, les différents dispositifs électriques médicaux implantés chez un patient doivent être connectés électriquement entre eux, par exemple pour avoir un système d'alimentation en énergie centralisé, ou pour que des données soient échangées entre les différents dispositifs.

Il existe donc un besoin pour faciliter les connexions électriques entre ces différents dispositifs implantés.

Le document US 4,411,276 décrit un dispositif implantable permettant une connexion entre fils électriques. Un but de la présente invention est donc de fournir un dispositif de connexion électrique destiné à être implanté à l'intérieur du corps d'un animal, tel qu'un humain, pour permettre une connexion électrique entre différents dispositifs électriques.

En particulier, un but de la présente invention est de fournir un dispositif de connexion électrique implantable dans le corps d'un animal, tel qu'un humain, permettant une connexion électrique fiable, à arrachement « taré », entre les dispositifs électriques tout en offrant une simplicité d'utilisation, notamment en termes de connexion des fils électriques entre eux.

### EXPOSE DE L'INVENTION

A cette fin, on propose un dispositif de connexion électrique destiné à être implanté à l'intérieur du corps d'un animal pour permettre une connexion électrique entre une pluralité de fils électriques, comprenant :
- un boîtier comprenant au moins un orifice formé à la surface dudit boîtier à l'extrémité d'au moins une cavité ménagée à l'intérieur du boîtier, ladite cavité étant destinée à recevoir un premier fil électrique à travers l'orifice ;
- des moyens d'interconnexion électrique pour établir une connexion électrique entre ledit premier fil électrique et au moins un deuxième fil électrique ;
- un moyen de maintien en position du premier fil électrique dans la cavité correspondante, ledit moyen de maintien en position comprenant une came montée en rotation dans le boîtier pour comprimer ledit premier fil électrique contre les parois intérieures de la cavité lors d'une rotation de la came dans un sens tendant à insérer ledit premier fil électrique vers l'intérieur de la cavité correspondante,
- le moyen de maintien en position comprenant une came montée en rotation dans le boîtier pour comprimer ledit premier fil électrique contre les parois intérieures de la cavité lors d'une rotation de la came dans un sens tendant à insérer ledit premier fil électrique vers l'intérieur de la cavité correspondante, et
- le moyen de maintien en position comprenant en outre un moyen de verrouillage pour verrouiller la came en position lorsque ladite came est en position de compression du premier fil électrique, le moyen de verrouillage comprenant un organe de verrouillage monté coulissant dans le boîtier et destiné à être inséré dans un rail complémentaire prévu dans la came jusqu'à venir en butée contre une extrémité fermée dudit rail de manière à empêcher tout mouvement de rotation de la came.

Des aspects préférés mais non limitatifs de ce dispositif de connexion électrique, pris seuls ou en combinaison, sont les suivants :
- la came a un profil avec une portion de compression adaptée pour comprimer le premier fil électrique contre les parois intérieures de la cavité, ladite portion de compression comprenant des moyens adaptés pour engager mécaniquement le premier fil électrique.
- la portion de compression est crantée, dentée, et/ou rugueuse.
- le moyen de maintien en position comprend un moyen d'actionnement pour mettre en rotation la came.
- le moyen de verrouillage comprend en outre un mécanisme à ressort destiné à bloquer automatiquement l'organe de verrouillage lorsqu'il est positionné en bout de course dans le rail complémentaire de la came.
- le dispositif comprend en outre des moyens d'étanchéité agencés au niveau de l'orifice formé dans le boîtier à l'extrémité de la cavité, de manière à isoler l'intérieur de la cavité de l'extérieur du boîtier.
- la surface du boîtier présente un décrochement en périphérie de l'orifice, ledit décrochement étant adapté pour coopérer avec un renflement prévu sur ledit premier fil de manière à former un joint d'étanchéité entre l'intérieur de la cavité et l'extérieur du boîtier lorsque ledit premier fil électrique est en position dans la cavité.
- le décrochement a une forme cylindrique, tronconique ou demi-sphérique.
- Le dispositif est caractérisé en ce que :
   ▪ le boîtier comprend un pluralité d'orifices formés à la surface dudit boîtier à l'extrémité de cavités ménagées à l'intérieur du boîtier, chaque cavité étant destinée à recevoir au moins un fil électrique ;
   ▪ les moyens d'interconnexion électrique sont agencés pour établir une connexion électrique déterminée entre les différents fils électriques ; et
   ▪ le dispositif comprend autant de moyens de maintien en position que d'orifices prévus à la surface du boîtier, les moyens de maintien associés à chaque orifice ayant une structure et un fonctionnement identiques.
- les cavités sont longitudinales et s'étendent selon un axe, les axes des différentes cavités étant contenus dans un seul et même plan, appelé plan de cavités, chaque came étant montée dans le boîtier en rotation par rapport à un axe de rotation normal au plan de cavités.

Selon un autre aspect de l'invention, on propose un ensemble de connexion électrique, caractérisé en ce qu'il comprend :
- un dispositif de connexion électrique comme décrit précédemment, et
- au moins un fil électrique destiné à être inséré dans l'orifice formé à la surface du boîtier du dispositif de connexion électrique, ledit fil électrique comprenant une portion de gaine présentant un renflement sur la totalité de la périphérie du fil électrique, ledit renflement étant agencé sur le fil électrique à une position spécifiquement choisie pour que le renflement prenne appui sur la surface du boîtier au niveau de l'orifice lorsque ledit fil électrique est en position dans la cavité.

Des aspects préférés mais non limitatifs de cet ensemble de connexion électrique sont les suivants :
- le renflement a une forme cylindrique, conique, ou demi-sphérique.
- le renflement est ménagé sur un manchon adapté pour être positionné autour du fil électrique.
- le renflement a une forme complémentaire du décrochement prévu à la surface du boîtier au niveau de l'orifice, de manière à former un joint d'étanchéité entre l'intérieur de la cavité et l'extérieur du boîtier lorsque ledit premier fil électrique est en position dans la cavité.

### DESCRIPTION DES FIGURES

D'autres caractéristiques et avantages de l'invention ressortiront encore de la description qui suit, laquelle est purement illustrative et non limitative et doit être lue en regard des dessins annexés, sur lesquels :
- la figure 1 est une représentation schématique du système de connexion électrique proposé ;
- la figure 2 est une représentation schématique d'un mode de réalisation du dispositif de connexion électrique présenté ;
- les figures 3 à 5 sont des représentations schématiques de différents modes de réalisation des moyens d'étanchéité pour le dispositif de connexion électrique de la figure 2.

### DESCRIPTION DETAILLEE DE L'INVENTION

Comme on l'a vu ci-dessus, il existe de plus en plus de dispositifs médicaux implantés nécessitant une alimentation électrique permanente par l'intermédiaire d'un câble de connexion électrique, jouant le rôle de câble d'alimentation électrique, placé en percutané et qui présente donc l'inconvénient de pouvoir s'infecter au niveau du passage percutané, cette infection pouvant se propager jusqu'au dispositif médical implanté, ce que l'on cherche à éviter.

Outre le fait qu'il existe un certain nombre de dispositifs médicaux implantés dont il est impossible de stopper le fonctionnement pendant un temps suffisamment long qui permettrait de remplacer le câble d'alimentation électrique infecté sans mettre en danger la vie du patient, il existe également un certain nombre de cas où le câble d'alimentation électrique ne peut être remplacé indépendamment du dispositif médical. Dans ces derniers cas, il est nécessaire, si l'on souhaite stopper la progression de l'infection, de remplacer l'ensemble médical complet composé du dispositif médical implanté du câble d'alimentation percutané, ce qui représente des coûts très élevés, tant au niveau du matériel à remplacer, qu'au niveau de l'intervention chirurgicale en tant que telle.

Pour résoudre cette problématique, on propose un système de connexion électrique nouveau qui présente la caractéristique d'être amovible par rapport au dispositif médical implanté, tout en présentant une voie d'alimentation électrique de secours parallèle à la voie d'alimentation électrique principale, cette voie d'alimentation électrique de secours permettant d'alimenter le dispositif médical implanté lorsque le câble principal d'alimentation électrique du système de connexion qui est infecté doit être enlevé pour être remplacé.

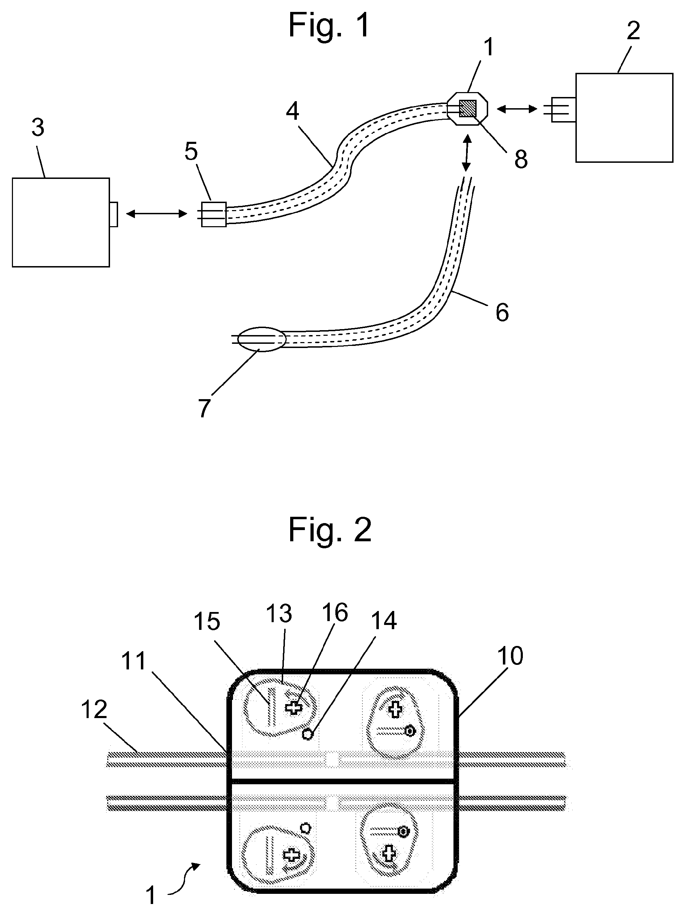

Comme cela est illustré schématiquement à la figure 1, le système de connexion électrique proposé comprend un câble d'alimentation électrique principal 4 comprenant un certain nombre de fils électriques permettant le passage de courant électrique entre un dispositif d'alimentation électrique 3 externe au corps humain et le dispositif électrique médical 2 implanté dans le corps du patient. Ce câble principal d'alimentation 4 a de préférence les même caractéristiques que le câble d'alimentation électrique classique, en particulier ce câble d'alimentation électrique principal 4 est adapté pour être placé en percutané pour relier électriquement le système d'alimentation électrique externe 3 au dispositif électrique médical implanté 2.

La particularité du système de connexion électrique proposé réside dans la connexion électrique entre le câble d'alimentation principal 4 et le dispositif médical implanté 2. En effet, l'extrémité du câble d'alimentation électrique principal 4 destinée à être connectée au dispositif électrique médical implanté 2 comprend un dispositif de connexion 1 particulier qui est adapté d'une part pour permettre une connexion électrique entre les fils électriques de même phase du câble électrique d'alimentation principal 4 et du dispositif électrique médical implanté 2, cette connexion se faisant par l'intermédiaire de connecteurs et de moyens d'interconnexion particuliers, et qui comprend d'autre part des connecteurs supplémentaires agencés pour permettre une connexion électrique entre des fils électriques du dispositif médical implanté 2 et des fils électriques externes au câble d'alimentation principal 4.

Le dispositif de connexion 1 placé à l'extrémité implantée du câble d'alimentation principal 4, et dont on présentera un mode de réalisation particulier plus loin dans ce texte, permet donc de pouvoir brancher un deuxième ensemble de fils électriques, assemblés par exemple dans un câble électrique secondaire 6, au dispositif médical implanté 2. En particulier, le système de connexion électrique proposé permet donc d'avoir une deuxième voie d'alimentation électrique en parallèle de la première voie d'alimentation électrique de manière à pouvoir alimenter en énergie le dispositif électrique médical implanté 2 par cette deuxième voie d'alimentation si cela s'avérait nécessaire.

Prenons l'exemple non limitatif du cas où le dispositif médical implanté est une pompe cardiaque, de type assistance ventriculaire gauche implantée (AVGI), pour laquelle l'alimentation électrique nécessairement permanente est habituellement effectuée par l'intermédiaire d'un câble d'alimentation électrique fixé de manière non amovible à la pompe, et couplé par voie percutanée à une source d'énergie externe au patient.

De telles pompes d'assistance cardiaque sont généralement implantées de manière temporaire chez le patient, mais les durées d'implantation sont aujourd'hui de plus en plus longues, de sorte qu'il n'est pas rare que le câble d'alimentation électrique se rompe ou s'infecte à partir du passage percutané correspondant. Dès que l'infection débute au niveau du passage percutané, l'infection se propage le long du câble d'alimentation en direction de la pompe d'assistance cardiaque, et il est donc impératif de pouvoir stopper cette propagation de l'infection avant qu'elle n'atteigne la pompe afin de préserver la vie du patient.

En utilisant une pompe d'assistance cardiaque associée à un système d'alimentation électrique sous la forme du système de connexion électrique proposé, il est possible de stopper la propagation de l'infection en remplaçant de manière relativement simple et peu coûteuse le câble principal d'alimentation électrique.

En effet dans ce cas, le dispositif électrique médical implanté 2, c'est-à-dire la pompe d'assistance cardiaque, est connecté à un câble d'alimentation électrique principal 4 par l'intermédiaire d'un dispositif de connexion électrique 1 particulier permettant la connexion d'un deuxième câble d'alimentation électrique 6 en parallèle du câble d'alimentation électrique principal 4. Ainsi, lorsque le câble d'alimentation électrique principal 4 est infecté il est possible de connecter une deuxième source d'alimentation électrique au dispositif électrique médical implanté par l'intermédiaire d'un deuxième câble d'alimentation électrique 6 connecté au dispositif de connexion électrique 1. Avec un tel système de connexion, il est donc possible de couper l'alimentation électrique principale tout en continuant d'alimenter en énergie la pompe d'assistance cardiaque par l'intermédiaire du deuxième câble d'alimentation électrique 6 couplé à une source d'énergie, garantissant ainsi le fonctionnement continu de la pompe.

Dès lors que la pompe d'assistance cardiaque est alimentée par une source d'alimentation électrique secondaire, il est possible de déconnecter le câble d'alimentation principal 4 du dispositif de connexion 1 de manière à le retirer du patient et à supprimer toute source d'infection. Le câble d'alimentation principal 4 peut donc être remplacé facilement sans mettre en danger la vie du patient par un arrêt du dispositif électrique médical implanté puisque ce dernier continue d'être alimenté par l'intermédiaire du deuxième câble d'alimentation électrique 6 connecté au dispositif de connexion électrique 1. Une fois que le câble d'alimentation électrique principal 4 infecté a été retiré, on peut en insérer un nouveau en conditions stériles, pour le connecter au dispositif de connexion électrique 1 d'une part et à une source d'alimentation électrique externe 3 d'autre part, de manière à ce que la pompe d'assistance cardiaque puisse de nouveau être alimentée en énergie par l'intermédiaire de ce câble d'alimentation électrique principal 4. Dans ce cas, le câble d'alimentation électrique « de secours » 6, placé pour les besoins de l'intervention, peut être retiré, et le passage percutané du câble d'alimentation électrique principal 4 refermé de manière à retrouver un fonctionnement standard du dispositif électrique médical implanté 2 par l'intermédiaire du câble d'alimentation électrique principal 4 connecté à une source d'alimentation électrique externe 3.

Pour être effectué dans des conditions de succès optimales, le changement du câble d'alimentation électrique principal 4 impose un protocole opératoire rigoureux répondant à plusieurs impératifs :
- effectuer dans un premier temps une connexion du câble d'alimentation électrique « de secours » 6 dans des conditions stériles, en pratiquant une voie d'abord chirurgicale aseptique au regard du dispositif de connexion 1 couplé au dispositif électrique médical implanté 2 ;
- retirer, dans un second temps, le câble d'alimentation électrique principal 4 infecté en le déconnectant du dispositif de connexion 1, et en le tractant vers l'extérieur de l'organisme tout en évitant une propagation de l'infection que l'on cherche à éradiquer.

Il est donc nécessaire de pratiquer un geste chirurgical nécessitant une asepsie très rigoureuse non loin d'une zone infectée par des germes non exceptionnellement résistants aux antibiotiques courants.

Selon un autre mode de réalisation de l'invention, le système de connexion électrique proposé est implanté, connecté d'une part au dispositif médical implanté 2 et d'autre part au dispositif d'alimentation électrique externe 3, par l'intermédiaire du câble électrique d'alimentation principal 4 relié au dispositif de connexion 1 proposé, le système de connexion comprenant en outre un câble d'alimentation électrique « de secours » 6 dont les fils électriques sont reliés aux connecteurs supplémentaires du dispositif de connexion 1 de manière à pouvoir être interconnectés avec les fils électriques de même phase du dispositif électrique médical implanté 2.

Dans ce cas là, le câble d'alimentation électrique « de secours » 6 est donc connecté à l'une de ses extrémités au dispositif de connexion 1, et est connecté à son autre extrémité à des moyens de connexion spécifiques 7 par l'intermédiaire desquels il est possible de relier ledit câble d'alimentation électrique « de secours » 6 à une source d'alimentation électrique externe au patient.

Cet ensemble composé du câble d'alimentation électrique « d'urgence » 6, des moyens de connexions électriques supplémentaires 7, associé au dispositif de connexion 1 présenté, constituent une connexion électrique complémentaire d'urgence (CECU).

Les moyens de connexions électriques complémentaires 7 placés à l'extrémité libre du câble d'alimentation électrique « d'urgence » 6 peuvent avoir des formes différentes. De préférence, on utilise des moyens de connexion destinés à être placés en sous-cutané de manière à pouvoir être accessibles facilement par un praticien depuis l'extérieur du patient sans avoir nécessairement à effectuer d'incisions spécifiques.

On peut par exemple utiliser un socle placé en sous-cutané et comprenant des connecteurs sous forme de fiches femelles dans lesquelles il est possible d'implanter des aiguilles connectées à une source d'alimentation électrique externe, et par l'intermédiaire desquelles l'électricité peut être conduite vers le câble d'alimentation électrique « de secours » 6.

De manière préférée, ces moyens de connexion sous-cutanés 7 sont agencés et positionnés chez le patient de manière à pouvoir être facilement accessibles depuis l'extérieur, et de manière fiable.

Le connecteur sous-cutané 7 peut par exemple avoir la forme d'un un boitier ouvert comprenant une pluralité de compartiments internes isolés électriquement les uns des autres, chaque compartiment étant rempli d'un matériau conducteur dans lequel des fiches de connexion électrique peuvent être insérées. Le matériau conducteur de chaque compartiment est en outre connecté à des fils électriques du câble d'alimentation électrique « d'urgence » 6. Par ailleurs, une membrane d'étanchéité vient fermer le boitier, ladite membrane d'étanchéité étant formée dans un matériau isolant et souple adapté pour une insertion des fiches de connexion électrique depuis l'extérieur du boitier à travers ladite membrane d'étanchéité vers les matériaux conducteurs des compartiments.

Outre le fait de pouvoir de gérer les problèmes de changement de câble d'alimentation électrique en cas d'infection issue du passage percutané, un tel mode de réalisation du système de connexion présentant un câble d'alimentation « de secours » 6 placé *in situ* permet également de pouvoir substituer rapidement une deuxième source d'alimentation électrique à la source d'alimentation électrique principale 3 en cas de défaillance subite de celle-ci, ou par exemple lorsque le câble d'alimentation électrique principal 4 est endommagé et ne permet plus d'alimenter en énergie le dispositif électrique médical implanté 2.

Comme il a déjà été mentionné, le système de connexion électrique proposé peut être adapté à tout type de dispositif électrique médical implanté nécessitant de préférence une alimentation électrique permanente.

Le système de connexion électrique proposé est particulièrement adapté pour les alimentations électriques de dispositifs électriques médicaux ayant pour but d'assurer le fonctionnement d'un organe défaillant, comme c'est le cas par exemple de la pompe d'assistance cardiaque présentée.

Le système de connexion électrique proposé peut également être utilisé pour d'autres types de dispositifs électriques médicaux implantés, tels que par exemple des appareils destinés à délivrer des substances médicamenteuses directement à l'intérieur du corps humain, de façon contrôlée.

Ce système de connexion électrique particulier peut également être utilisé afin d'alimenter électriquement un dispositif implanté de génération d'énergie, un tel dispositif de génération d'énergie électrique étant en général connecté à plusieurs autres dispositifs médicaux implantés à l'intérieur du corps du patient.

En outre, le système de connexion électrique proposé peut être utilisé pour connecter électriquement tout type dispositif électrique interne, c'est-à-dire implanté chez le patient, à tout type de dispositif électrique externe, y compris des dispositifs de recueil de données mesurées à l'intérieur du corps du patient.

Le dispositif de connexion électrique 1 proposé comprend des moyens d'interconnexions électriques 8 particuliers permettant de relier les fils électriques du câble principal 4 aux fils électriques de même phase du dispositif électrique médical implanté 2 d'une part, et de relier les fils électriques d'un éventuel câble d'alimentation électrique « de secours » 6 aux fils électriques de même phase du dispositif électrique médical implanté 2 d'autre part. Ces moyens d'interconnexions 8 peuvent avoir une structure plus ou moins complexe.

Selon une forme la plus simple, l'interconnexion des différents connecteurs est réalisée par un câblage par soudure. Selon un mode de réalisation plus complexe, les moyens d'interconnexions sont réalisés par l'intermédiaire d'un circuit imprimé qui permet d'associer les connecteurs entre eux de manière à relier les fils électriques de même phase selon un schéma particulier, défini en fonction des besoins identifiés.

Selon un mode de réalisation particulier, les moyens d'interconnexion 8 peuvent être actionnés par l'intermédiaire d'un sélecteur qui permet de modifier les connexions électriques, par exemple pour relier soit le câble d'alimentation électrique principal 4, soit le câble d'alimentation électrique « de secours » 6, au dispositif électrique médical implanté 2. Dans ce cas, une diode peut par exemple indiquer quel port électrique est fonctionnel.

Le dispositif de connexion électrique 1 utilisé dans le système de connexion électrique proposé à l'extrémité du câble d'alimentation électrique principal 4 peut prendre différentes formes pour peu que ce dispositif de connexion électrique 1 soit adapté pour une implantation à l'intérieur du corps humain, et qu'il permette une connexion électrique fiable entre différents fils électriques issus de dispositifs ou de câbles différents tout en proposant une connexion amovible de ces différents fils électriques.

En effet, pour le système de connexion électrique présenté ci-dessus, il est impératif de pouvoir avoir un dispositif de connexion 1 qui permet une connexion amovible et fiable du câble d'alimentation électrique principal 4, ainsi qu'une connexion amovible, et la plus fiable possible, de l'éventuel câble d'alimentation électrique secondaire 6, dit câble « de secours ».

Selon un mode de réalisation particulier, tel qu'illustré dans la figure 2, on propose un dispositif de connexion électrique 1 se présentant sous la forme d'un boîtier 10 qui comprend un certain nombre de cavités internes à l'intérieur desquelles il est possible d'insérer des fils électriques 12 par l'intermédiaire d'orifices 11 ménagés à la surface du boîtier 10. Par ailleurs, le dispositif de connexion électrique 1 comprend des moyens d'interconnexion (non représentés) qui sont adaptés pour établir une connexion électrique spécifique entre les différents fils électriques 12 insérés dans le dispositif de connexion électrique 1, de manière en particulier à relier entre eux les fils électriques 12 de même phase prévus pour une alimentation électrique.

Il est à noter que le boîtier 10 peut avoir une forme quelconque, par exemple parallélépipédique ou ovoïdale. En outre le boîtier est de préférence conçu dans un matériau biocompatible, ainsi que tout élément le composant pouvant être amené à être en contact avec l'intérieur du corps du patient.

Comme mentionnée plus haut, il est nécessaire que le dispositif de connexion électrique 1 présenté soit adapté pour une connexion amovible des fils électriques 12, tout en garantissant un maintien en position fiable du fil électrique 12 dans la cavité correspondante lorsque l'on ne souhaite pas que ce fil électrique 12 soit déconnecté du dispositif de connexion électrique 1.

A cette fin, le boîtier 10 intègre donc des moyens de maintien en position 13, de préférence au niveau de chaque orifice, de manière permettre un maintien en position du fil électrique 12 inséré à travers l'orifice 11 correspondant. Plus précisément, le moyen de maintien en position proposé comprend une came 13 montée en rotation dans le boîtier 10 de manière à comprimer le fil électrique 12, lorsque celui-ci est inséré à travers l'orifice 11 correspondant, contre les parois intérieures de la cavité.

La came 13, montée en rotation dans le boîtier 10, a donc une portion de compression qui est adaptée pour comprimer le fil électrique 12 contre les parois intérieures de la cavité correspondante lorsque la came 13 est dans une position particulière, et une surface dite surface neutre, qui n'exerce aucune pression sur le fil électrique 12 lorsqu'elle est en regard de ce dernier.

La came 13 est montée en rotation dans le boîtier 10 de sorte qu'une rotation de la came 13 dans un sens tendant à insérer le fil électrique 12 vers l'intérieur de la cavité amène la surface de compression de la came en regard dudit fil électrique 12 de manière à le comprimer contre la paroi interne de la cavité correspondante.

Ainsi, lorsque la came 13 est actionnée en rotation de manière à venir comprimer le fil électrique correspondant, le mouvement de rotation de la came 13 tend à insérer le fil à l'intérieur du boîtier 10 ce qui est préférable pour la fiabilité de la connexion électrique au sein du dispositif de connexion électrique 1.

Le moyen de maintien en position comprend en outre un moyen de verrouillage (14 ;15) destiné à verrouiller la came 13 lorsqu'elle est en position de compression du fil électrique 12. Ce moyen de verrouillage (14 ; 15) permet donc d'empêcher que le fil électrique 12 puisse être retiré du boîtier 10 de manière involontaire.

Un tel moyen de verrouillage (14 ; 15) comprend de préférence un organe de verrouillage 14, monté par exemple coulissant à l'intérieur du boîtier 10, et étant adapté pour coopérer avec un rail complémentaire15 prévu dans la came 13 de maintien en position. Lorsque la came 13 est tournée de sorte que la portion de compression vienne comprimer le fil électrique 12 contre la paroi intérieure de la cavité, l'organe de verrouillage 14 vient s'insérer à l'intérieur du rail complémentaire 15 prévu dans la came 13. L'organe de verrouillage 14 coulisse à l'intérieur du rail complémentaire 15 prévu dans la came 13 jusqu'à venir en butée contre l'extrémité fermée du rail complémentaire 15 au niveau duquel est prévu un mécanisme permettant de verrouiller l'organe de verrouillage 14 en position dans le rail complémentaire 15. Ce mécanisme peut par exemple être un mécanisme à ressort agencé pour être automatiquement activé lorsque l'organe de verrouillage 14 se place dans une position spécifique à l'intérieur du rail complémentaire 15, par exemple en butée contre l'extrémité fermée dudit rail complémentaire 15.

Ainsi, lorsque la came 13 est dans une position pour comprimer le fil électrique 12 contre les parois intérieures de la cavité, cette came 13 est maintenue verrouillée en position grâce au moyen de verrouillage (14 ; 15) décrit. Cela permet d'empêcher que le fil électrique 12 soit retiré du dispositif de connexion 1, ce qui garantit une connexion électrique fiable entre les différents fils électriques 12. S'il s'avérait nécessaire de retirer ledit fil électrique 12, il suffit de désactiver le moyen de verrouillage (14 ; 15) en désenclenchant par exemple le mécanisme à ressorts le cas échéant.

La surface de compression de la came 13 peut être adaptée pour effectuer un engagement mécanique du fil électrique 12 destiné à être comprimé. Ainsi, la portion de compression de la came 13 peut par exemple être crantée, ou dentée, et/ou rugueuse, de sorte que la surface de compression puisse venir en engagement à l'intérieur de la gaine prévue autour du fil électrique 12. Une telle surface de compression crantée ou dentée de la came 13 permet non seulement un meilleur engagement du fil électrique 12 au sein de la cavité du boîtier 10, mais permet également à la came 13 d'être adaptée pour des fils électriques ayant des diamètres légèrement différents. En effet, ces différences de diamètre peuvent être compensées par la profondeur d'insertion des crans ou des dents à l'intérieur de la gaine entourant le fil électrique 12.

Par ailleurs, le dispositif de connexion électrique 1 peut être adapté pour que l'insertion des fils électriques 12 dans le boîtier 10 se fasse de manière étanche, c'est-à-dire que l'intérieur des cavités ménagées dans le boîtier 10 soit isolé du milieu extérieur au boîtier 10 lorsque les fils électriques 12 sont en position, connectés dans ledit dispositif de connexion électrique 1. A cette fin, il est préférable de réaliser l'étanchéité mentionnée au niveau de chacun des orifices 11 ménagés dans le boîtier 10.

Le boîtier 10 peut par exemple présenter un décrochement 111 aux périphéries de l'orifice 11, ledit décrochement étant adapté pour coopérer avec un renflement 121 prévu sur le fil électrique 12 de sorte que la coopération entre le renflement 121 et le décrochement 111 forment un joint d'étanchéité entre l'intérieur de la cavité et l'extérieur du boîtier 10 lorsque le fil électrique 12 est en position connectée à l'intérieur de la cavité.

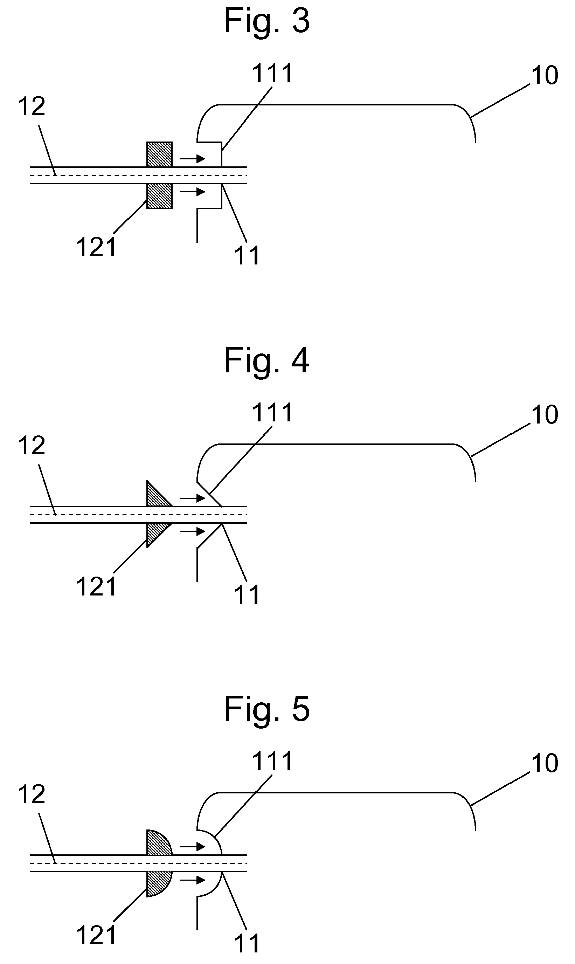

Les figures 3, 4 et 5 illustrent des modes de réalisation particuliers d'un tel agencement d'étanchéité par la coopération d'un décrochement 111 ménagé au niveau de l'orifice 11 du boîtier 10 avec un renflement 121 prévu sur le fil électrique 12 destiné à être inséré à travers ledit orifice 11.

Comme illustré sur la figure 3, le décrochement 111 peut avoir une forme cylindrique, le renflement 121 prévu autour du fil électrique 12 ayant alors également une forme cylindrique autour du fil, complémentaire du décrochement cylindrique 111 ménagé au niveau de la périphérie de l'orifice 11 du boîtier.

Selon un autre mode de réalisation tel qu'illustré à la figure 4, le décrochement 111 ménagé au niveau de l'orifice 11 a une forme tronconique adaptée pour coopérer avec un renflement 121 ayant une forme d'anneau également tronconique entourant le fil 12.

On peut également prévoir un décrochement 111 au niveau de l'orifice 11 ayant une forme demi-sphérique adaptée pour coopérer avec un renflement 121 ménagé autour du fil électrique 12 et ayant également une forme demi-sphérique adaptée pour coopérer avec la forme demi-sphérique du décrochement 111.

Il est à noter que ces deux derniers modes de réalisation sont particulièrement performants pour garantir une étanchéité certaine entre l'intérieur de la cavité et l'extérieur du boîtier 10 puisque l'étanchéité sera assurée même si il existe des imperfections au moment de la formation du décrochement 111 dans le boîtier et/ou du renflement 121 au niveau du fil électrique 12.

On pourrait également envisager un mode de réalisation dans lequel la surface du boîtier 10 au niveau de l'orifice 11 ne présente aucun décrochement, auquel cas l'étanchéité entre l'intérieur de la cavité et l'extérieur du boîtier 10 est assurée par le seul renflement 121 prévu au niveau du fil 12 qui vient s'appuyer sur l'orifice 11 et garantit ainsi une étanchéité certaine. Dans ce cas, le renflement 121 peut avoir également une forme quelconque, de préférence demi-sphérique, tronconique, ou encore cylindrique.

Le renflement 121 prévu sur le fil électrique 12 peut être directement formé dans la gaine isolante entourant ledit fil électrique 12. Selon un mode de réalisation préféré, le renflement 121 prévu pour entourer le fil électrique 12 est formé dans un manchon qui est adapté pour entourer la gaine isolante du fil électrique 12 de sorte qu'il est possible de positionner ledit renflement 121 à n'importe quel niveau par rapport à l'extrémité du fil électrique 12 que l'on souhaite connecter au dispositif de connexion 1. Cela est d'autant plus avantageux que la position du renflement 121 par rapport à l'orifice 11 est critique pour que l'étanchéité entre l'intérieur de la cavité et l'extérieur du boîtier 10 soit la plus efficace possible. De manière encore préférée, la came 13, destinée à maintenir en position le fil électrique 12 à l'intérieur de la cavité, est adaptée pour coopérer avec le manchon portant le renflement 121 autour du fil 12, de sorte que la came 13 permette de maintenir en position non seulement le fil électrique 12, mais également le renflement 121 lui correspondant.

Par ailleurs, la came 13 comprend des moyens d'actionnement 16 qui permettent de mettre ladite came 13 dans la position désirée, pour maintenir le fil électrique 12 fixé à l'intérieur du boîtier 10 par exemple, ou pour libérer ce fil électrique 12. Ce moyen d'actionnement 16 pourra par exemple prendre la forme d'une encoche spécifique à l'intérieur de laquelle il est possible de glisser un outil depuis l'extérieur du boîtier 10, comme par exemple un tournevis, de sorte que la rotation de la came 13 à l'intérieur du boîtier 10 soit effectuée de manière mécanique, par un praticien, ce qui garantit encore une meilleure fiabilité au dispositif de connexion électrique 1 présenté.

Si le dispositif de connexion électrique 1 a été présenté en référence au système de connexions électriques décrit plus haut, son utilisation n'est bien évidemment pas limitée au système de connexion électrique décrit. En particulier, un tel dispositif de connexion électrique 1 peut être utilisé pour coupler entre eux des dispositifs électriques médicaux implantés à l'intérieur du corps du patient, ou encore pour permettre une connexion électrique entre une source d'alimentation en énergie implantée et un ou plusieurs dispositifs électriques médicaux implantés, etc.

En outre, le dispositif de connexion électrique 1 présenté a été décrit en référence avec une seule cavité et un seul orifice prévus pour la connexion d'un seul fil électrique. Il va s'en dire que l'enseignement technique correspondant peut être adapté pour former un dispositif de connexion électrique 1 comprenant une pluralité de cavités, une pluralité d'orifices, et une pluralité de moyens de maintien en position des fils destinés à être insérés à l'intérieur de chaque orifice, les moyens d'interconnexion prévus à l'intérieur du dispositif de connexion électrique 1 étant adaptés en fonction des besoins de connexion électrique que l'on souhaite réaliser par l'intermédiaire du dispositif de connexion électrique 1 présenté.

De la même façon, le système de connexion électrique a été présenté en relation avec la seule problématique d'alimentation électrique d'un dispositif électrique médical implanté. Il pourrait toutefois être adapté à d'autres fins, par exemple pour garantir une transmission continue de données recueillies par le dispositif médical implanté ou de données commandant ce dispositif médical implanté. De manière préférée, le système de connexion électrique présenté est utilisé pour assurer à la fois une alimentation électrique continue, mais également pour permettre une transmission fiable de données particulières, ce qui impose simplement d'adapter le nombre de fils électriques intégrés dans les différents câbles, et le nombre de connecteurs du dispositif de connexion.

## Revendications

1. Dispositif de connexion électrique (1) destiné à être implanté à l'intérieur du corps d'un animal pour permettre une connexion électrique entre une pluralité de fils électriques (12), comprenant :
- un boîtier (10) comprenant au moins un orifice (11) formé à la surface dudit boîtier (10) à l'extrémité d'au moins une cavité ménagée à l'intérieur du boîtier (10), ladite cavité étant destinée à recevoir un premier fil électrique (12) à travers l'orifice (11) ;
- des moyens d'interconnexion électrique (8) pour établir une connexion électrique entre ledit premier fil électrique (12) et au moins un deuxième fil électrique (12);
- un moyen de maintien en position du premier fil électrique (12) dans la cavité correspondante,
**caractérisé en ce que** ledit moyen de maintien en position comprend une came (13) montée en rotation dans le boîtier (10) pour comprimer ledit premier fil électrique (12) contre les parois intérieures de la cavité lors d'une rotation de la came (13) dans un sens tendant à insérer ledit premier fil électrique (12) vers l'intérieur de la cavité correspondante, et **en ce que** ledit moyen de maintien en position comprend en outre un moyen de verrouillage (14 ;15) pour verrouiller la came (13) en position lorsque ladite came (13) est en position de compression du premier fil électrique (12), le moyen de verrouillage comprenant un organe de verrouillage (14) monté coulissant dans le boîtier (10) et destiné à être inséré dans un rail complémentaire (15) prévu dans la came (13) jusqu'à venir en butée contre une extrémité fermée dudit rail (15) de manière à empêcher tout mouvement de rotation de la came (13).

2. Dispositif (1) selon la revendication 1, **caractérisé en ce que** la came (13) a un profil avec une portion de compression adaptée pour comprimer le premier fil électrique (12) contre les parois intérieures de la cavité, ladite portion de compression comprenant des moyens adaptés pour engager mécaniquement le premier fil électrique.

3. Dispositif (1) selon la revendication 2, **caractérisé en ce que** la portion de compression est crantée, dentée, et/ou rugueuse.

4. Dispositif (1) selon l'une quelconque des revendications 1 à 3, **caractérisé en ce que** le moyen de maintien en position comprend un moyen d'actionnement (16) pour mettre en rotation la came (13).

5. Dispositif (1) selon l'une quelconque des revendications 1 à 4, **caractérisé en ce que** le moyen de verrouillage (14 ;15) comprend en outre un mécanisme à ressort destiné à bloquer automatiquement l'organe de verrouillage (14) lorsqu'il est positionné en bout de course dans le rail complémentaire (15) de la came (13).

6. Dispositif (1) selon l'une quelconque des revendications 1 à 5, **caractérisé en ce qu'**il comprend en outre des moyens d'étanchéité agencés au niveau de l'orifice (11) formé dans le boîtier (10) à l'extrémité de la cavité, de manière à isoler l'intérieur de la cavité de l'extérieur du boîtier (10).

7. Dispositif (1) selon la revendication 6, **caractérisé en ce que** la surface du boîtier (10) présente un décrochement (111) en périphérie de l'orifice (11), ledit décrochement (111) étant adapté pour coopérer avec un renflement (121) prévu sur ledit premier fil (12) de manière à former un joint d'étanchéité entre l'intérieur de la cavité et l'extérieur du boîtier (10) lorsque ledit premier fil électrique (12) est en position dans la cavité.

8. Dispositif (1) selon la revendication 7, **caractérisé en ce que** le décrochement (111) a une forme cylindrique, tronconique ou demi-sphérique.

9. Dispositif (1) selon l'une quelconque des revendications 1 à 8, **caractérisé en ce que** :
- le boîtier (10) comprend un pluralité d'orifices (11) formés à la surface dudit boîtier (10) à l'extrémité de cavités ménagées à l'intérieur du boîtier (10), chaque cavité étant destinée à recevoir au moins un fil électrique (12);
- les moyens d'interconnexion électrique (8) sont agencés pour établir une connexion électrique déterminée entre les différents fils électriques (12); et
- le dispositif (1) comprend autant de moyens de maintien en position que d'orifices (11) prévus à la surface du boîtier (10), les moyens de maintien associés à chaque orifice (11) ayant une structure et un fonctionnement identiques.

10. Dispositif (1) selon la revendication 9, **caractérisé en ce que** les cavités sont longitudinales et s'étendent selon un axe, les axes des différentes cavités étant contenus dans un seul et même plan, appelé plan de cavités, chaque came (13) étant montée dans le boîtier (10) en rotation par rapport à un axe de rotation normal au plan de cavités.

11. Ensemble de connexion électrique, **caractérisé en ce qu'**il comprend :
- un dispositif de connexion électrique (1) selon l'une quelconque des revendications 1 à 10, et
- au moins un fil électrique (12) destiné à être inséré dans l'orifice (11) formé à la surface du boîtier (10) du dispositif (1) de connexion électrique, ledit fil électrique (12) comprenant une portion de gaine présentant un renflement (121) sur la totalité de la périphérie du fil électrique (12), ledit renflement (121) étant agencé sur le fil électrique (12) à une position spécifiquement choisie pour que le renflement (121) prenne appui sur la surface du boîtier (10) au niveau de l'orifice (11) lorsque ledit fil électrique (12) est en position dans la cavité.

12. Ensemble selon la revendication 11, **caractérisé en ce que** le renflement (121) a une forme cylindrique, conique, ou demi-sphérique.

13. Ensemble selon l'une quelconque des revendications 11 et 12, **caractérisé en ce que** le renflement (121) est ménagé sur un manchon adapté pour être positionné autour du fil électrique (12).

14. Ensemble selon la revendication 11 prise en combinaison avec la revendication 7, **caractérisé en ce que** le renflement (121) a une forme complémentaire du décrochement (111) prévu à la surface du boîtier (10) au niveau de l'orifice (11), de manière à former un joint d'étanchéité entre l'intérieur de la cavité et l'extérieur du boîtier (10) lorsque ledit premier fil électrique (12) est en position dans la cavité.

## Patentansprüche

1. Elektrische Verbindungsvorrichtung (1) zur Implantation in den Körper eines Tiers, um die elektrische Verbindung zwischen einer Vielzahl elektrischer Drähte (12) zu erlauben, umfassend:
- ein Gehäuse (10), das mindestens eine auf der Oberfläche des Gehäuses (10) am Ende von mindestens einem in dem Gehäuse (10) ausgebildeten Hohlraum ausgebildete Öffnung (11) umfasst, wobei der Hohlraum zur Aufnahme eines ersten elektrischen Drahts (12) durch die Öffnung (11) bestimmt ist,
- elektrische Verbindungsmittel (8) zur Herstellung einer elektrischen Verbindung zwischen dem ersten elektrischen Draht (12) und mindestens einem zweiten elektrischen Draht (12),
- ein Positionshaltemittel des ersten elektrischen Drahts (12) in dem entsprechenden Hohlraum,
**dadurch gekennzeichnet, dass** das Positionshaltemittel einen Nocken (13) umfasst, der rotierend in dem Gehäuse (10) montiert ist, um den ersten elektrischen Draht (12) gegen die Innenwände des Hohlraums bei einer Rotation des Nockens (13) in eine Richtung zu komprimieren, bei welcher der erste elektrische Draht (12) in das Innere des entsprechenden Hohlraums befördert wird, und dass das Positionshaltemittel ferner ein Verriegelungsmittel (14; 15) zum Verriegeln des Nockens (13) in Position umfasst, wenn sich der Nocken (13) in Kompressionsposition des ersten elektrischen Drahts (12) befindet, wobei das Verriegelungsmittel ein Verriegelungsorgan (14) umfasst, das gleitend in dem Gehäuse (10) montiert ist und zum Einsetzen in eine in dem Nocken (13) vorgesehene komplementäre Schiene (15) bis zum Anschlag an einem geschlossenen Ende der Schiene (15) bestimmt ist, um jedwede Rotationsbewegung des Nockens (13) zu blockieren.

2. Vorrichtung (1) nach Anspruch 1, **dadurch gekennzeichnet, dass** der Nocken (13) ein Profil mit einem Kompressionsabschnitt hat, der ausgebildet ist, um den ersten elektrischen Draht (12) gegen die Innenwände des Hohlraums zu komprimieren, wobei der Kompressionsabschnitt Mittel umfasst, die ausgebildet sind, um den ersten elektrischen Draht mechanisch einzusetzen.

3. Vorrichtung (1) nach Anspruch 2, **dadurch gekennzeichnet, dass** der Kompressionsabschnitt geriffelt, gezahnt und/oder rau ist.

4. Vorrichtung (1) nach einem der Ansprüche 1 bis 3, **dadurch gekennzeichnet, dass** das Positionshaltemittel ein Betätigungsmittel (16) umfasst, um den Nocken (13) in Rotation zu versetzen.

5. Vorrichtung (1) nach einem der Ansprüche 1 bis 4, **dadurch gekennzeichnet, dass** das Verriegelungsmittel (14; 15) ferner einen Federmechanismus umfasst, der bestimmt ist, das Verriegelungsorgan (14) automatisch zu blockieren, wenn es am Ende des Wegs in der komplementären Schiene (15) des Nockens (13) positioniert ist.

6. Vorrichtung (1) nach einem der Ansprüche 1 bis 5, **dadurch gekennzeichnet, dass** sie ferner Dichtungsmittel umfasst, die im Bereich der Öffnung (11) ausgebildet sind, die in dem Gehäuse (10) am Ende des Hohlraums ausgebildet sind, um das Innere des Hohlraums von außerhalb des Gehäuses (10) zu isolieren.

7. Vorrichtung (1) nach Anspruch 6, **dadurch gekennzeichnet, dass** die Oberfläche des Gehäuses (10) einen Rücksprung (111) am Umfang der Öffnung (11) aufweist, wobei der Rücksprung (111) ausgebildet ist, um mit einer Verdickung (121) auf dem ersten Draht (12) zusammenzuarbeiten, um eine Dichtung zwischen dem Innern des Hohlraums und außerhalb des Gehäuses (10) zu bilden, wenn der erste elektrische Draht (12) in dem Hohlraum positioniert ist.

8. Vorrichtung (1) nach Anspruch 7, **dadurch gekennzeichnet, dass** der Rücksprung (111) eine zylindrische, kegelstumpfförmige oder halbkugelige Form hat.

9. Vorrichtung (1) nach einem der Ansprüche 1 bis 8, **dadurch gekennzeichnet, dass**:
- das Gehäuse (10) eine Vielzahl von Öffnungen (11) auf der Oberfläche des Gehäuses (10) am Ende von in dem Gehäuse (10) ausgebildeten Hohlräumen hat, wobei jeder Hohlraum zur Aufnahme von mindestens einem elektrischen Draht (12) bestimmt ist,
- die elektrischen Verbindungsmittel (8) ausgebildet sind, um eine bestimmte elektrische Verbindung zwischen den verschiedenen elektrischen Drähten (12) herzustellen, und
- die Vorrichtung (1) ebenso viele Positionshaltemittel umfasst, wie Öffnungen (11) auf der Oberfläche des Gehäuses (10) vorgesehen sind, wobei die jeder Öffnung (11) zugeordneten Positionshaltemittel eine identische Struktur und Funktion haben.

10. Vorrichtung (1) nach Anspruch 9, **dadurch gekennzeichnet, dass** die Hohlräume länglich sind und sich gemäß einer Achse erstrecken, wobei die Achsen der verschiedenen Hohlräume in einer einzigen und selben, als Hohlraumebene bezeichneten Ebene enthalten sind, wobei jeder Nocken (13) in dem Gehäuse (10) in Rotation im Verhältnis zu einer zur Ebene des Hohlraums normalen Rotationsachse montiert ist.

11. Elektrische Verbindungseinheit, **dadurch gekennzeichnet, dass** sie umfasst:
- eine elektrische Verbindungsvorrichtung (1) nach einem der Ansprüche 1 bis 10, und
- mindestens einen elektrischen Draht (12), der zum Einsetzen in die auf der Oberfläche des Gehäuses (10) der elektrischen Verbindungsvorrichtung (1) ausgebildeten Öffnung (11) bestimmt ist, wobei der elektrische Draht (12) einen Mantelabschnitt umfasst, der eine Verdickung (121) auf dem gesamten Umfang des elektrischen Drahts (12) aufweist, wobei die Verdickung (121) auf dem elektrischen Draht (12) an einer speziell gewählten Stelle ausgebildet ist, damit sich die Verdickung (121) auf der Oberfläche des Gehäuses (10) im Bereich der Öffnung (11) abstützt, wenn der elektrische Draht (12) in dem Hohlraum in Position ist.

12. Einheit nach Anspruch 11, **dadurch gekennzeichnet, dass** die Verdickung (121) eine zylindrische, konische oder halbkugelige Form hat.

13. Einheit nach einem der Ansprüche 11 und 12, **dadurch gekennzeichnet, dass** die Verdickung (121) auf einer Muffe ausgebildet ist, die für die Positionierung um den elektrischen Draht (12) geeignet ist.

14. Einheit nach Anspruch 11, herangezogen in Kombination mit Anspruch 7, **dadurch gekennzeichnet, dass** die Verdickung (121) eine zum auf der Oberfläche des Gehäuses (10) im Bereich der Öffnung (11) vorgesehenen Rücksprung (111) komplementäre Form hat, so dass eine Dichtung zwischen dem Innern des Hohlraums und außerhalb des Gehäuses (10) gebildet ist, wenn der erste elektrische Draht (12) im Hohlraum in Position ist.

## Claims

1. An electrical connection device (1) designed to be implanted inside an animal's body to enable an electrical connection between several electrical wires (12), comprising:
- a housing (10) comprising at least one opening (11) formed at the surface of said housing (10) at the end of at least one cavity formed inside the housing (10), said cavity being designed to receive a first electrical wire (12) through the opening (11);
- electrical interconnection means (8) to establish an electrical connection between said first electrical wire (12) and at least a second electrical wire (12);
- a position maintenance means for maintaining the position of the first electrical wire (12) in the corresponding cavity,
**characterized in that** said position maintenance means comprises a cam (13) rotatably mounted in the housing (10) to compress said first electrical wire (12) against the inner walls of the cavity during rotation of the cam (13) in a direction tending to insert said first electrical wire (12) inside the corresponding cavity, and **in that** said position maintenance means further comprises a locking means (14; 15) to lock the cam (13) in position when said cam (13) is positioned to compress the first electrical wire (12), the locking means comprising a locking component (14) slidably mounted in the housing (10) and designed to be inserted into a complementary rail (15) provided in the cam (13) until it comes to stop against a closed end of said rail (15) so as to prevent any rotation movement of the cam (13).

2. The device (1) according to claim 1, **characterized in that** the cam (13) has a profile with a compression portion designed to compress the first electrical wire (12) against the inner walls of the cavity, said compression portion comprising means designed to mechanically engage the first electrical wire.

3. The device (1) according to claim 2, **characterized in that** the compression portion is serrated, toothed, and/or rough.

4. The device (1) according to any one of claims 1 to 3, **characterized in that** the position maintenance means comprises an actuator means (16) to rotate the cam (13).

5. The device (1) according to any one of claims 1 to 4, **characterized in that** the locking means (14; 15) further comprises a spring mechanism designed to automatically lock the locking component (14) when it is positioned at the end of course in the complementary rail (15) of the cam (13).

6. The device (1) according to any one of claims 1 to 5, **characterized in that** it further comprises sealing means arranged at the opening (11) formed in the housing (10) at the end of the cavity so as to insulate the inside of the cavity from the outside of the housing (10).

7. The device (1) according to claim 6, **characterized in that** the surface of the housing (10) has a recess (111) in the periphery of the opening (11), said recess (111) being designed to cooperate with a protrusion (121) provided on said first wire (12) so as to form a sealing joint between the inside of the cavity and the outside of the housing (10) when said first electrical wire (12) is in position in the cavity.

8. The device (1) according to claim 7, **characterized in that** the recess (111) has a cylindrical, truncated or hemispherical shape.

9. The device (1) according to any one of claims 1 to 8, **characterized in that**:
- the housing (10) comprises several openings (11) formed at the surface of said housing (10) at the end of the cavities formed inside the housing (10), each cavity being designed to receive at least one electrical wire (12);
- the electrical interconnection means (8) are arranged to establish a determined electrical connection among the various electrical wires (12); and
- the device (1) comprises as many position maintenance means as openings (11) provided at the surface of the housing (10), the maintenance means associated with each opening (11) having an identical structure and function.

10. The device (1) according to claim 9, **characterized in that** the cavities are longitudinal and extend along an axis, the axes of the various cavities being contained in a same single plane, called cavity plane, each cam (13) being rotatably mounted in the housing (10) around a normal axis of rotation of the cavity plane.

11. An electrical connection assembly, **characterized in that** it comprises:
- an electrical connection device (1) according to any one of claims 1 to 10, and
- at least one electrical wire (12) designed to be inserted into the opening (11) formed at the surface of the housing (10) of the electrical connection device (1), said electrical wire (12) comprising a sheath portion with a protrusion (121) over the entire periphery of the electrical wire (12), said protrusion (121) being arranged on the electrical wire (12) at a position specifically chosen so that the protrusion (121) rests on the surface of the housing (10) at the opening (11) when said electrical wire (12) is in position in the cavity.

12. The assembly according to claim 11, **characterized in that** the recess (121) has a cylindrical, truncated or hemispherical shape.

13. The assembly according to any one of claims 11 to 12, **characterized in that** the protrusion (121) is formed on a sleeve designed to be positioned around the electrical wire (12).

14. The assembly according to claim 11 taken in combination with claim 7, **characterized in that** the protrusion (121) has a shape complementary to the recess (111) provided at the surface of the housing (10) at opening (11), so as to form a sealing joint between the inside of the cavity and the outside of housing (10) when said first electrical wire (12) is in position in the cavity.
